# EUROPEAN PATENT APPLICATION

(11) **EP 1 449 483 A1**
(43) Date of publication of application: **25.08.2004**
(21) Application number: 02783633.7
(22) Date of filing: 27.11.2002
(51) Int. Cl.: A61B 10/00

(54) **JUDGMENT ABILITY EVALUATION APPARATUS, ROBOT, JUDGMENT ABILITY EVALUATION METHOD, PROGRAM, AND MEDIUM**

(30) Priority: 28.11.2001 JP 2001363199
(71) Applicant: MATSUSHITA ELECTRIC INDUSTRIAL CO., LTD., Kadoma-shi, Osaka 571-8501 (JP)
(72) Inventor: YAMAMOTO, Hiroshi, Shijonawate-shi, Osaka 575-0013 (JP); OHTA, Kazuyo, Katano-shi, Osaka 576-0053 (JP)
(74) Representative: Grünecker, Kinkeldey, Stockmair & Schwanhäusser Anwaltssozietät
(86) International application number: PCT/JP2002/012350
(87) International publication number: WO 2003/045252

(57) **Abstract**

There are intellectual evaluation devices in which interview content is previously registered by a doctor, its answers are managed electronically, and intellectual activity is evaluated for preventing the decline of mental activity such as dementia. However, with such conventional intellectual evaluation devices, the declining degree of intellectual activity of the user could not be monitored properly, for example, for the reasons that the subject feels resistance to being subjected to judgment evaluation, and the like.

A judgment evaluation device 100 comprising an output means 102 of outputting a question to the user, an input means 104 of inputting the user's answer to the output question, a judging means 105 of determining whether or not the input answer is correct, and an evaluation means 106 of evaluating the user's judgment based on the result of determination.

## Description

### Technical Field

The present invention relates to a judgment evaluation device, robot, judgment evaluation method, program, and medium which can be used in homes for elderly people, hospitals, home, and the like.

### Background Art

It is thought that "improvement in diet," "moderate exercise," and "having hobbies" are effective for preventing the decline of mental activity such as dementia.

When attention is paid to hobbies among these, it is thought that those done by using hands and fingers, such as "handicrafts" and "gardening," those requiring thinking, such as "go" and "shogi," and those stimulating five senses, such as doing exercise while listening to "music," are effective.

On the other hand, there is an intellectual evaluation device in which interview content is previously registered by a doctor, its answers are managed electronically, and intellectual activity is evaluated (for example, refer to Japanese Unexamined Patent Application No. H08-117210).

The entire disclosure of Japanese Unexamined Patent Application No. H08-117210 is incorporated herein by citation (reference) in its entirety.

However, with such conventional intellectual evaluation devices, the declining degree of intellectual activity could not be monitored properly, for example, for the reasons that the subject feels resistance to being subjected to judgment evaluation, and the like.

In addition, for mild dementia, for example, the cases where the person himself is conscious of it are few, and rehabilitation is not done willingly in some cases, so that, the inventor analyzes, methods that require complicated operation and the assistance of others can not readily be carried out, therefore in some cases continuous use can not be expected.

### Disclosure of the Invention

In view of such conventional problems as described above, it is an object of the present invention to provide a judgment evaluation device, robot, judgment evaluation method, program, and medium that can monitor the declining degree of intellectual activity more properly, for example, without making a subj ect feel resistance to being subj ected to judgment evaluation.

A first invention of the present invention is a judgment evaluation device (100) comprising:
a question output means (102) of outputting a question to a user;
an answer input means (104) of inputting an answer of the user to the output question;
a determining means (105) of determining whether or not the input answer is correct; and
a judgment evaluation means (106) of evaluating a judgment of the user based on a result of the determination.

A second invention of the present invention is the judgment evaluation device (100) according to the first invention of the present invention, further comprising:
a question making means (109) of making the output question, and
a correct answer making means (103) of making a correct answer for the made question,
wherein the determining means (105) determines whether or not the input answer is correct based on the made correct answer.

A third invention of the present invention is the judgment evaluation device (100) according to the second invention of the present invention, further comprising an answer content recording means (107) of recording a content of the answer,
wherein the question making means (109) makes the output question based on the recorded answer content.

A fourth invention of the present invention is the judgment evaluation device (100) according to the second invention of the present invention, wherein the question making means (109) makes the question based on a result of the evaluation.

A fifth invention of the present invention is the judgment evaluation device (100b) according to the second invention of the present invention, further comprising a sensor means (304) of detecting a behavior of the user,
wherein the question making means (109) makes the output question based on a result of the detection, and
the correct answer making means (103) makes a correct answer for the made question based on the result of the detection.

A sixth invention of the present invention is the judgment evaluation device (800) according to the second invention of the present invention, further comprising an input recognition means (802) of recognizing a predetermined input from the user, and a response generating means (803) of generating a predetermined response to the user based on a result of the recognition,
wherein the question making means (109) makes the output question based on the input recognition and/or the response generation.

A seventh invention of the present invention is the judgment evaluation device (100) according to the first invention of the present invention, wherein the evaluation means (106) evaluates the judgment of the user by considering at least one of (1) date and time when the question is output to the user and/or date and time when the answer of the user to the question is input, and (2) a difficulty of the question output to the user.

An eighth invention of the present invention is the judgment evaluation device (100) according to the first , invention of the present invention, wherein the question output means (120) periodically outputs the question based on a predetermined standard.

A ninth invention of the present invention is the judgment evaluation device (100) according to the first invention of the present invention, wherein the question output means (102) outputs the question using voice synthesis, and
the answer input means (104) inputs the answer using voice recognition.

A tenth invention of the present invention is the judgment evaluation device (100) according to the first invention of the present invention, further comprising an evaluation-result-information-notifying-to-outside means (102) of notifying information regarding a result of the evaluation to outside.

An eleventh invention of the present invention is a judgment evaluation device (900) comprising:
a question output means (901) of outputting substantially the same question to a user for a plurality of times;
an answer input means (901) of inputting an answer of the user to the substantially the same, question output for the plurality of times; and
a judgment evaluation means (903) of evaluating a judgment of the user based on variation in answers of the user to the substantially the same question.

A twelfth invention of the present invention is the judgment evaluation device (900) according to the eleventh invention of the present invention, wherein the evaluation is evaluation regarding a degree of dementia of the user.

A thirteenth invention of the present invention is the' judgment evaluation device (900) according to the twelfth invention of the present invention, further comprising:
a user personal data holding means (902) of holding user personal data regarding a personal voice and/or personal image of the user;
a user person recognition means (903) of performing user person recognition regarding the personal voice and/or personal image of the user; and
a question making means (903) of making the output question using the held user personal data based on a result of the performed user person recognition.

A fourteenth invention of the present invention is a robot (800) further comprising the judgment evaluation device (100) according to any of the first to the thirteenth inventions of the present invention; and
a condition changing means (803) of changing a condition based on a result of the evaluation.

A fifteenth invention of the present invention is the robot (800) according to the fourteenth invention of the present invention, wherein the changing a condition is changing a condition regarding at least one of utterance, expression, and gesture.

A sixteenth invention of the present invention is the robot (800) according to the fifteenth invention of the present invention, further comprising a user presence sensing means (904) of sensing a presence of the user,
wherein the condition changing means (803) changes the condition based on a result of the sensing as well when a question is output to the user.

A seventeenth invention of the present invention is a judgment evaluation method comprising:
a question output step of outputting a question to a user;
an answer input step of inputting an answer of the user to the output question;
a determining step of determining whether or not the input answer is correct; and
a judgment evaluation step of evaluating a judgment of the user based on a result of the determination.

An eighteenth invention of the present invention is a judgment evaluation method comprising:
a question output step of outputting substantially the same question to a user for a plurality of times;
an answer input step of inputting an answer of the user to the substantially the same question output for the plurality of times; and
a judgment evaluation step of evaluating a judgment of the user based on variation in answers of the user to the substantially the same question.

A nineteenth invention of the present invention is a program of causing a computer to perform the question output step, the answer input step, the determining step, and the judgment evaluation step of the judgment evaluation method according to the seventeenth invention of the present invention.

A twentieth invention of the present invention is a program of causing a computer to perform the question output step, the answer input step, and the judgment evaluation step of the judgment evaluation method according to the eighteenth invention of the present invention.

A twenty-first invention of the present invention is a medium supporting the program according to the nineteenth invention of the present invention, which can be processed by a computer.

A twenty-second invention of the present invention is a medium supporting the program according to the twentieth invention of the present invention, which can be processed by a computer.

### Brief Description of the Drawings

Fig. 1 is a configuration view of the judgment evaluation device 100 of Embodiment 1 according to the present invention.
Fig. 2 is an explanation chart of the data structure of the result recording means 107 of Embodiment 1 according to the present invention.
Fig. 3 is a configuration view of the evaluation means 106 of Embodiment 1 according to the present invention.
Fig. 4 is a configuration view of the judgment evaluation device 100a of Embodiment 2 according to the present invention.
Fig. 5 is an explanation chart of the time of question asking intervals in Embodiment according to the present invention.
Fig. 6 is a configuration view of the judgment evaluation device 100b of Embodiment 3 according to the present invention.
Fig. 7 is a configuration view of the judgment evaluation device 100c of Embodiment 4 according to the present invention.
Fig. 8 is a configuration view of the pet-type terminal 800 of Embodiment 5 according to the present invention.
Fig. 9 is a configuration view of the pet-type terminal 800a of Embodiment 6 according to the present invention.
Fig. 10 is a configuration view of the pet-type robot 900 of Embodiment 7 according to the present invention.
Fig. 11 is an explanation chart of the user personal data of Embodiment 7 according to the present invention.
Fig. 12 is an explanation chart of the conversation history data of Embodiment 7 according to the present invention.

### (Explanation of References)

101, 101a, 101b, 101c question making means
102, 102a, 102b, 102d output means
103, 103b correct answer making means
104, 104a, 104b, 104d input means
105, 105a, 105b judging means
106, 106a, 106b evaluation means
107, 107a, 107b result recording means
108, 108a, 108b question making element database
109, 109a, 109b, 109c question composing means
110 difficulty changing means
301, 301b correct answer percentage calculating means
302, 302b period calculating means
303 intellectual evaluation means
701 calculation symbol number deciding means
702 calculation symbol type deciding means
800 pet-type terminal
801 timer means
802 voice recognition means
803, 803a response generating means
901 mechanism
902 response parameter database
903 response emotion deciding means
904 control means
905 information notifying means

### Best Mode of Carrying Out the Invention

Embodiments according to the present invention will be described below with reference to the drawings.

### (Embodiment 1)

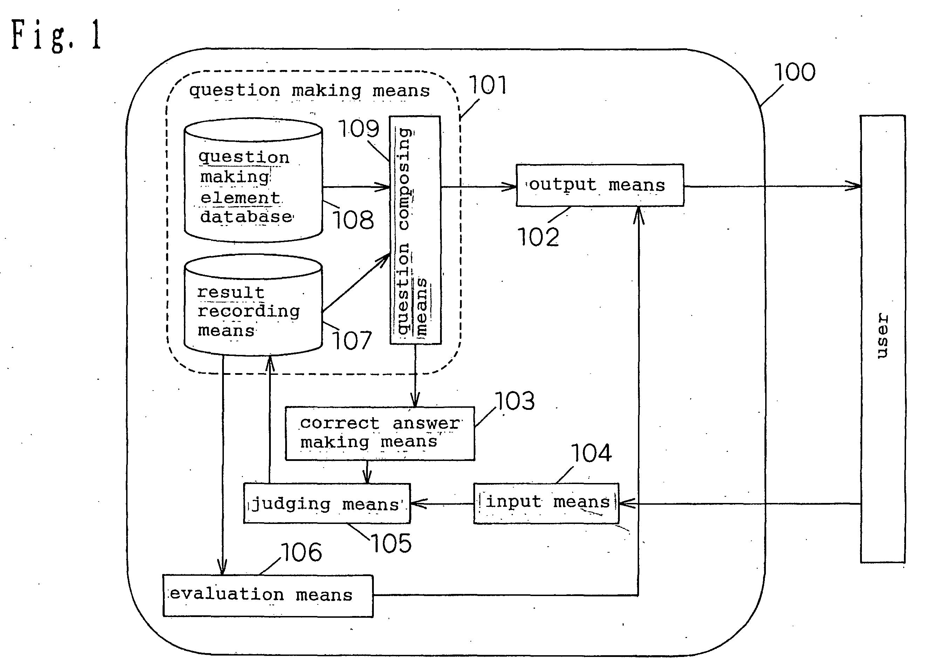

First, referring mainly to Fig. 1, a configuration view of the judgment evaluation device 100 of Embodiment 1 according to the present invention, the structure and operation of the judgment evaluation device 100 of this embodiment is described.

While the operation of the judgment evaluation device 100 of this embodiment is described, one embodiment of the intellectual judgment and mental function evaluation method of the present invention is described (the same applies hereinafter).
The judgment evaluation device 100 comprises a question making means 101, an output means 102, a correct answer making means 103, an input means 104, a judging means 105 and an evaluation means 106. Also, the question making means 101 comprises a result recording means 107, a question making element database 108, and a question composing means 109.

Here, a case where a calculation question is made by the question making means 101 is described.

It is desirable that a question to be made is at a level that general adult can answer promptly by mental calculation. For example, "5+4+6=?" can be made. Numbers which are the elements of a calculation question, the symbols of four operations, symbols which represent the end of a question such as "=?", and the like are held as a database in the question making element database 108, and the question composing means 109 reads each element from the question making element database 108 and combines numbers, operation symbols, and symbols which represent the end of a question, in this order, so as to make a question.

Here, while the symbols are held in the question making element database 108, of course, words such as "add, " "subtract, " and "-is?," which have the same meaning as the symbols, may be held.

The output means 102 is a CRT device and displays a question made by the question making means 101. Also, the correct answer making means 103 calculates the result of a calculation expression received from the question making means 101.

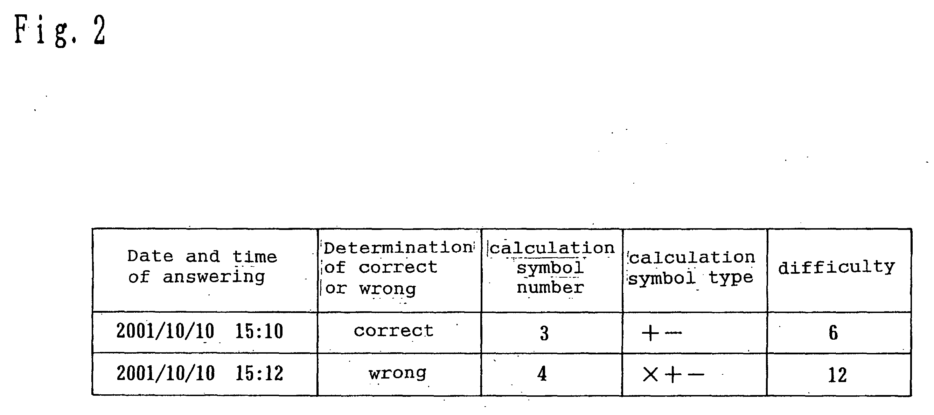

The input means 104 is rows of buttons which can input numbers, such as a keyboard, and the user can input a calculation result by pushing the buttons. The judging means 105 compares a result calculated by the correct answer making means 103 and an input value from the input means 104, performs judgment of being correct or wrong, and records the result in the result recording means 107. The difficulty of the question is also recorded in the result recording means 107 at the same time. The difficulty of the question is decided by the number, type, and the like of calculation symbols and is set to, for example, a value calculated from multiplication of the number of calculation symbols used for the question and the number of types of the calculation symbols. In this case, the larger the value of difficulty is, the more difficult the question is. An example of recorded data is shown in Fig. 2.

For example, for the question "3+5+4-6=?," the number of calculation symbols (calculation symbol number) is "3," and the number of types of the calculation symbols (calculation symbol type) is "2," thus, the difficulty of the same question is "6," which is the product of these. Also, for the question "2x3+5+4-6=?," the number of calculation symbols (calculation symbol number) is "4," and the number of types of the calculation symbols (calculation symbol type) is "3," thus, the difficulty of the same question is "12," which is the product of these. Of course, since it is thought, for example, that the difficulty of multiplication is higher than that of addition and subtraction, this may be considered to perform calculation of a difficulty.

The evaluation means 106 evaluates intellectual judgment from the results recorded in the result recording means 107.

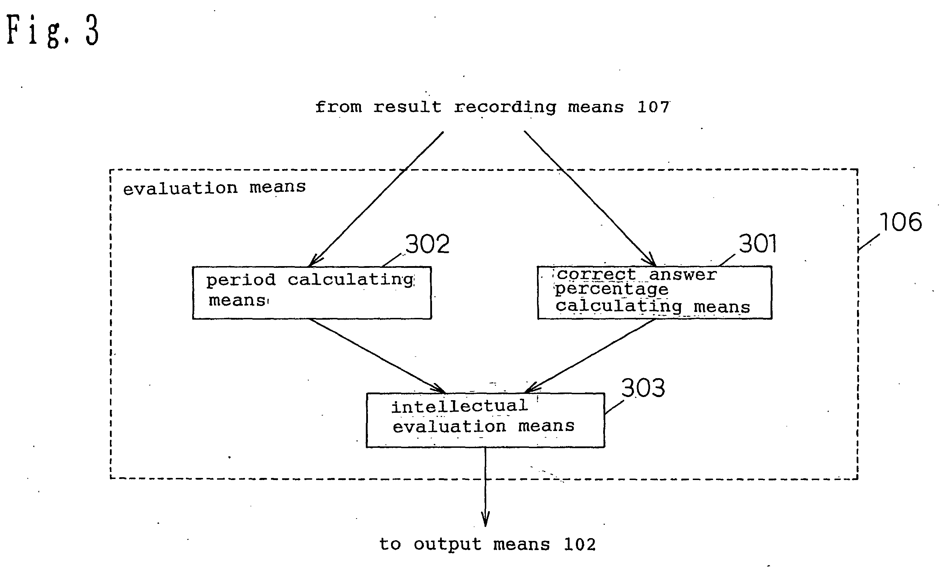

Fig. 3 is a configuration view of the evaluation means 106, in which 301 is a correct answer percentage calculating means, 302 is a period calculating means, and 303 is an intellectual evaluation means. The correct answer percentage calculating means 301 extracts recently asked questions having the same degree of difficulty which are recorded in the result recording means 107, calculates the percentage of correct answering from the answer results, and outputs the correct answer percentage to the intellectual evaluation means 303. Further, the period calculating means 302 calculates the period from the date and time when the questions, for which the correct answer percentage is calculated by the correct answer percentage calculating means 301, were asked to the present, and outputs the period to the intellectual evaluation means 303. The intellectual evaluation means 303 evaluates intellectual judgment from the results of the correct answer percentage calculating means 301 and the period calculating means 302.

For example, it is practical that the evaluation of intellectual judgment is higher as a correct answer can be given faster to a question having a higher difficulty. Such more practical evaluation can be performed by mounting the correct answer percentage calculating means 301 and the period calculating means 302.

The result of intellectual judgment may be output as CG (computer graphics) by the output means 102 to the user, or it may be output to the outside medical center, caregiver, and the like.

In Embodiment 1, it is desirable that the output means 102 comprises not only the CRT device but also a voice synthesis device and a voice output device, and outputs questions also by means of voice. Also, it is desirable that the input means 104 comprises not only the keyboard but also a voice input device and a voice recognition device and that the user can answer by means of voice.

As described above, using the judgment evaluation device in this Embodiment 1, the user uses the memory function of his brain for memorizing questions asked and the calculation function of his brain for actually performing calculation, so that while the evaluation of intellectual judgment is performed the effect of preventing the deterioration of intellectual judgment also can be expected.

The output means 102 corresponds to means including the question output means of the present invention, the input means 104 corresponds to means including the answer input means of the present invention, the judging means 105 corresponds to means including the determining means of the present invention, and the evaluation means 106 corresponds to means including the judgment evaluation means of the present invention. Also, the question composing means 109 corresponds to means including the question making means of the present invention, and the correct answer making means 103 corresponds to means including the correct answer making means of the present invention. Also, the result recording means 107 corresponds to means including the answer content recording means of the present invention. Also, the judgment evaluation device 100 corresponds to the judgment evaluation device of the present invention.

### (Embodiment 2)

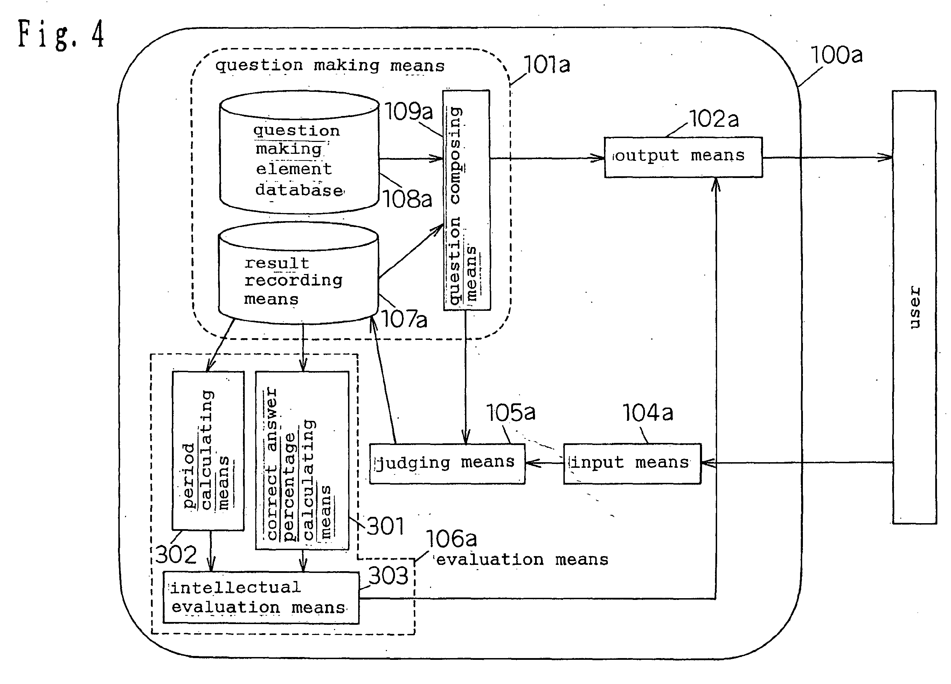

Next, referring mainly to Fig. 4, a configuration view of the judgment evaluation device 100a of Embodiment 2 according to the present invention, the structure and operation of the judgment evaluation device 100a of this embodiment is described.

In Fig. 4, an input means 104a is a plurality of colored buttons. For example, it consists of three buttons, "red," "blue," and "green."

A question making means 101a comprises a result recording means 107a, a question making element database 108a, and a question composing means 109a, and the words "red", "blue," and "green", which are the elements of a question, and the values of the time of question asking intervals are recorded and held in the question making element database 108a. The question composing means 109a makes a combination of colors recorded in the question making element database 108a and, further, outputs the combination to an output means 102a according to the time of a selected question asking interval.

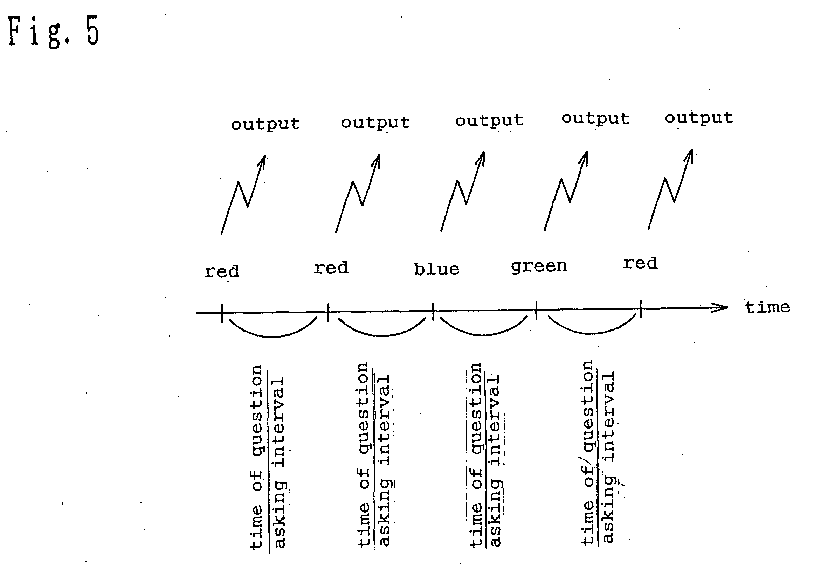

Voice output is performed, for example, as "red, red, blue, green, red." Here, the time of the question asking interval is the time after output of one color until output of the next color, as shown in Fig. 5.

The user pushes down the color buttons of the input means 104a in the same order as the question. A judging means 105a judges whether or not the order of the colors of the question is equal to the order of colors input by the input means 104a and records the result in the result recording means 107a. The difficulty of the question is also recorded in the result recording means 107a at the same time. The difficulty of the question is decided, for example, by the number of colors of the question asked and the number of changes of the colors.

An evaluation means 106a evaluates intellectual judgment from the result recorded in the result recording means 107a.

The correct answer percentage calculating means 301 extracts recently asked questions having the same degree of difficulty which are recorded in the result recording means 107a, calculates the percentage of correct answering from the answer results, and outputs the correct answer percentage to the intellectual evaluation means 303. Further, the period calculating means 302 calculates the period from the date and time when the question, for which the correct answer percentage is calculated by the correct answer percentage calculating means 301, was asked to the present, and outputs the period to the intellectual evaluation means 303. The intellectual evaluation means 303 evaluates intellectual judgment from the results of the correct answer percentage calculating means 301 and the period calculating means 302. The result of the intellectual judgment'is notified to the user through the output means 102a.

As described above, using the judgment evaluation device in this Embodiment 2, the user uses his memory function for memorizing color order and his movement function of pushing the color buttons, so that while the evaluation of intellectual judgment is performed the effect of preventing the deterioration of intellectual judgment also can be expected.

The output means 102a corresponds to means including the question output means and the evaluation-result-information-notifying-to-outside means of the present invention, the input means 104a corresponds to means including the answer input means of the present invention, the judging means 105a corresponds to means including the determining means of the present invention, and the evaluation means 106a corresponds to means including the judgment evaluation means of the present invention. Also, the question composing means 109a corresponds to means including the question making means and the correct answer making means of the present invention. Also, the result recording means 107a corresponds to means including the answer content recording means of the present invention. Also, the judgment evaluation device 100a corresponds to the judgment evaluation device of the present invention.

### (Embodiment 3)

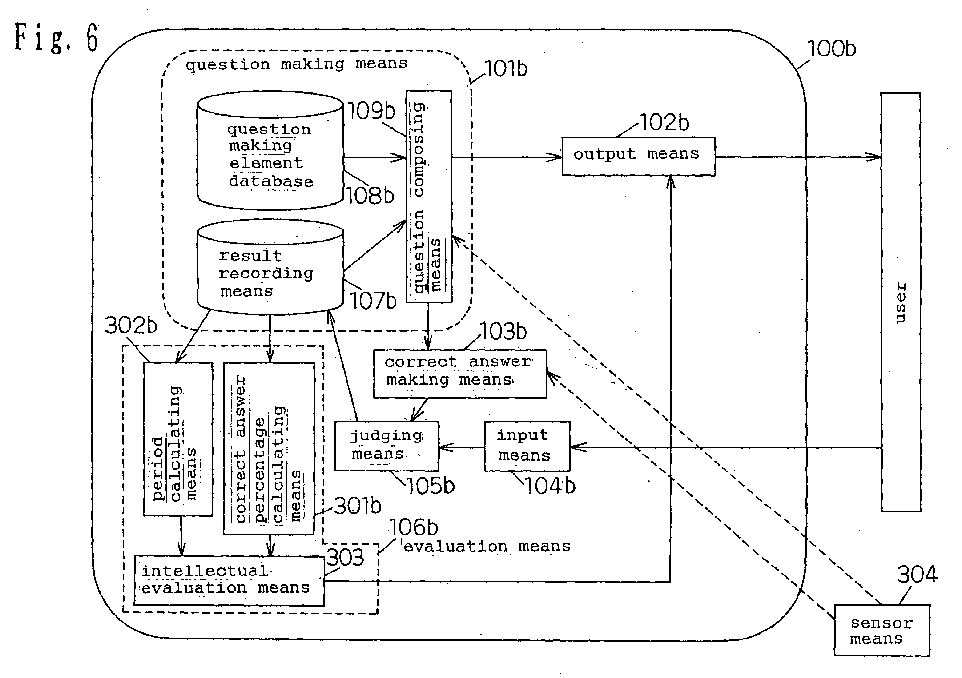

Next, referring mainly to Fig. 6, a configuration view of a judgment evaluation device 100b of Embodiment 3 according to the present invention, the structure and operation of the judgment evaluation device 100b of this embodiment is described.

In Fig. 6, a question making means 101b comprises a result recording means 107b, a question making element database 108b, and a question composing means 109b, and the words which are elements for making questions about content related to the user's behavior and personal information, such as "Did you go to the toilet?," "Did you have dinner?," and "When is your birthday?," which are question elements, are'recorded and held in the question making element database 108b, for example, the words "toilet," "dinner," "breakfast," "Did you go to?," and "Did you have?" are recorded and held. The question composing means 109b makes a combination of element words recorded in the question making element database 108b and outputs the combination to an output means 102b. Thus, voice outputs, for example, "Did you go to the toilet?" and "Did you have breakfast?," are performed.

A correct answer making means 103b previously registers correct personal information in the case where questions are related to personal information, and registers as correct an answer which was obtained when the most recent and the same question was asked, in the case where questions are related to behavior.

For example, in the case where a subject answers "Yes." to the question "Have you finished a meal?" which is performed at 13:00, "Yes." is registered as the correct answer to this question for the following 5 hours. Therefore, for example, in the case where the same subject answers "No." to the question "Have you finished a meal?" which is performed at 14:15, this answer is judged as incorrect.

Also, information as to whether the subject went to the toilet or not, and the like, is obtained from a sensor means 304 having a human body detecting sensor or a sensor of detecting water flushing, which is placed in the toilet or the like.

The user pushes down a button of an input means 104b, which corresponds to "Yes.," "No.," or the like, for answering a question. A judging means 105b judges whether or not the input answer is equal to the correct answer made by the correct answer making means 103b and records the result in the result recording means 107b.

An evaluation means 106b evaluates intellectual judgment from the result recorded in the result recording means 107b. A correct answer percentage calculating means 301b extracts recently asked questions which are recorded in the result recording means 107b, calculates the percentage of correct answering, and outputs the correct answer percentage to the intellectual evaluation means 303. Further, the period calculating means 302 calculates the period from the date and time when the question, for which the correct answer percentage is calculated by the correct answer percentage calculating means 301b, was asked to the present, and outputs the period to the intellectual evaluation means 303. The intellectual evaluation means 303 evaluates intellectual judgment from the results of the correct answer percentage calculating means 301b and the period calculating means 302, based on the assumption that "intellectual judgment is low when the question asking period is short and the correct answer percentage is low" and that "intellectual judgment is high when the question asking period is long and the correct answer percentage is high."

For example, the case where, although a subject who finished a meal at 12:30 returned the correct answer "Yes." to the question "Have you finished a meal?" which was performed at 13:00, the same subj ect returned the incorrect answer "No." to a question of the same content which was performed at 13:01, is the case where "although the question asking period is 1 minute and is short, the correct answer percentage is low." In this manner, the correct answer for a question, which is made based on a recorded history, may be made.

The result of intellectual judgment may be indicated as a numerical value such as "a numerical value obtained by dividing a correct answer percentage by a question asking period" or may be indicated as a relative expression such as "decreased" or "increased," and is notified to the user through the output means 102b.

As described above, using the judgment evaluation device in this Embodiment 3, memory function associated with the user's behavior is used, so that the evaluation of intellectual judgment can be performed.

The output means 102b corresponds to means including the question output means and the evaluation-result-information-notifying-to-outside-means of the present invention, the input means 104b corresponds to means including the answer input means of the present invention, the judging means 105b corresponds to means including the determining means of the present invention, and the evaluation means 106b corresponds to means including the judgment evaluation means of the present invention. Also, the question composing means 109b corresponds to means including the question making means of the present invention, and the correct answer making means 103b corresponds to means including the correct answer making means of the present invention. Also, the result recording means 107b corresponds to means including the answer content recording means of the present invention. Also, the sensor means 304 corresponds to means including the sensor means of the present invention. Also, the judgment evaluation device 100b corresponds to the judgment evaluation device of the present invention.

### (Embodiment 4)

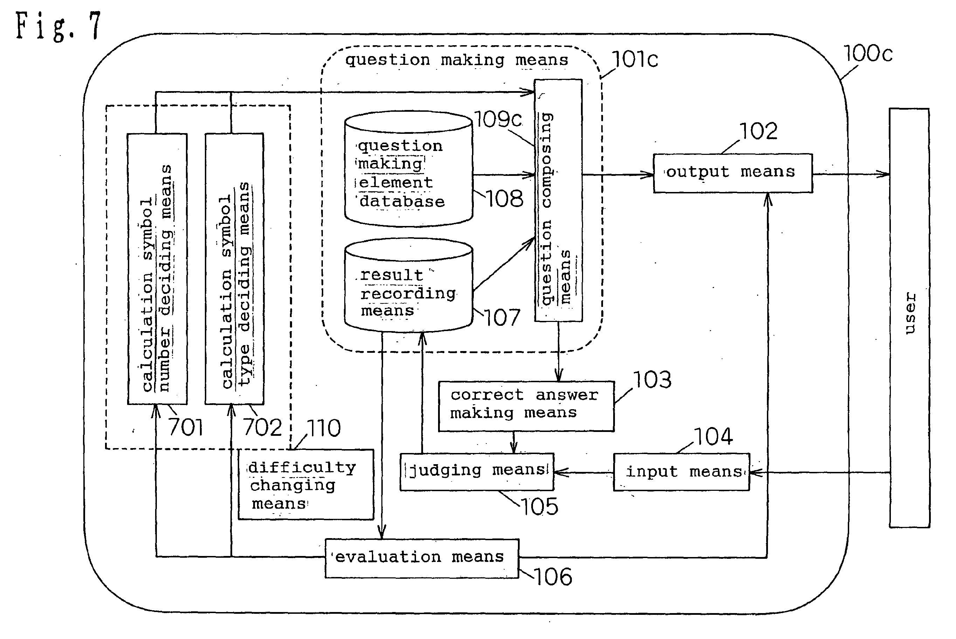

Next, referring mainly to Fig. 7, a configuration view of the judgment evaluation device 100c of Embodiment 4 according to the present invention, the structure and operation of the judgment evaluation device 100c of this embodiment is described.

The judgment evaluation device 100c comprises a question making means 101c, a difficulty changing means 110, an output means 102, a correct answer making means 103, an input means 104, a judging means 105, and an evaluation means 106, and the difficulty changing means 110 comprises a calculation symbol number deciding means 701 and a calculation symbol type deciding means 702.

Means having the same numbers denoted to as in Fig. 1 provide the same'operation as in Embodiment 1, and therefore their description is omitted.

The difficulty changing means 110 receives the previous evaluation results of intellectual judgment from the evaluation means 106, changes the difficulty of a question to be proposed to the user based on the received results, and outputs the difficulty to the question making means 101c. A method of changing and deciding a difficulty is, for example, previously preparing question settings which are effective for deterioration of intellectual judgment and the degrees of the deterioration and making a difficulty correspond in the question settings.

The calculation symbol number deciding means 701 increases the number of calculation symbols when the deterioration of intellectual judgment is judged as small in the result of the evaluation means 106, and, on the contrary, decreases the number of calculation symbols when the deterioration of intellectual judgment is judged as large. This is, for example, increasing the number of items of addition when the degree of dementia is small, and decreasing the number of items of addition when the condition is serious. Further, the calculation symbol type deciding means 702 increases the types of calculation symbols when the deterioration of intellectual judgment is judged as small in the result of the evaluation means 106, and, on the contrary, decreases the number of types of calculation symbols when the deterioration of intellectual judgment is judged as large. This is, for example, increasing the degree of mixing addition and subtraction when the degree of dementia is small, and' decreasing the mixing degree when the condition is serious.

In the question making means 101c, a question composing means 109c uses the type and number of calculation symbols, which are decided by the calculation symbol number deciding means 701 and the calculation symbol type deciding means 702, to make a question similarly as described in Embodiment 1.

For the rest, as in Embodiment 1, the judging means 105 judges whether or not a correct answer calculated by the correct answer making means 103 is equal to an answer input from the input means 104, and the result recording means 107 records its result.

As described above, by having the structure of this Embodiment 4, a question having a difficulty that is appropriate for each user can be asked, and as a result, continuous use can be expected.

While this Embodiment 4 is described in the form of extending Embodiment 1, of course, similar difficulty setting is also possible in Embodiment 2.

The output means 102 corresponds to means including the question output means and the evaluation-result-information-notifying-to-outside means of the present invention, the input means 104 corresponds to means including the answer input means of the present invention, the judging means 105 corresponds to means including the determining means of the present invention, and the evaluation means 106 corresponds to means including the judgment evaluation means of the present invention. Also, the question making means 101c and the difficulty changing means 110 correspond to means including the question making means of the present invention, and the correct answer making means 103 corresponds to means including the correct answer making means of the present invention. Also, the result recording means 107 corresponds to means including the answer content recording means of the present invention. Also, the judgment evaluation device 100c corresponds to the judgment evaluation device of the present invention.

### (Embodiment 5)

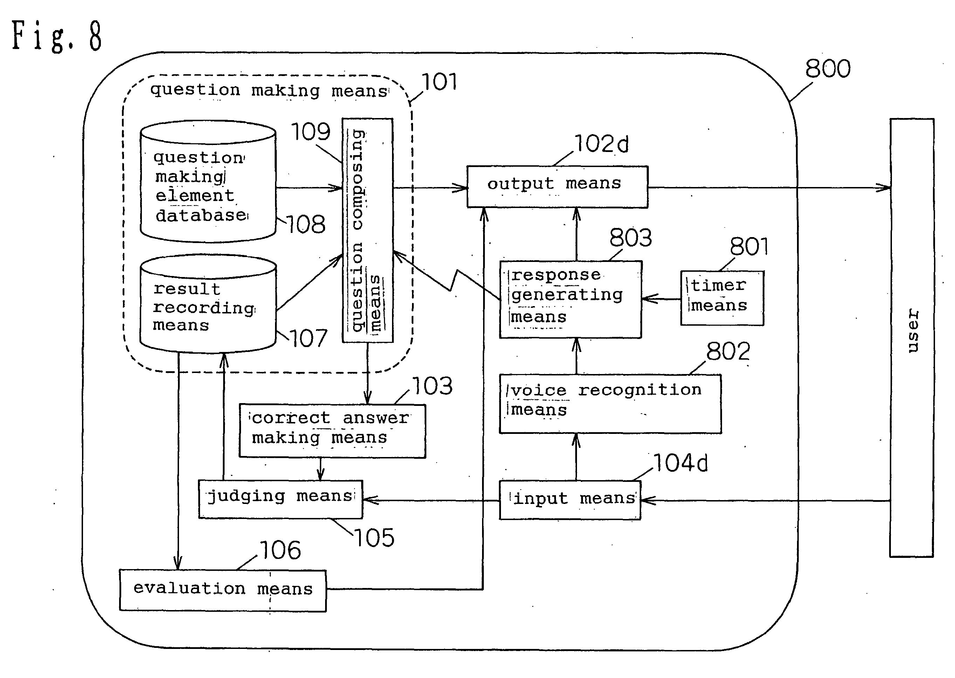

Next, referring mainly to Fig. 8, a configuration view of the pet-type terminal (intellectual judgment and mental function evaluation enabling pet-type robot) 800 that is Embodiment 5 according to the present invention, the structure and operation of the pet-type terminal 800 of this embodiment is described.

In Fig. 8, the pet-type terminal 800 has the shape of a stuffed toy mocking an animal or the like. An input means 104d of inputting the voice of the user is attached to its chest.

Originally, the pet-type terminal 800 is intended for providing various information through natural conversation, in which a voice recognition means 802 recognizes voice input from the input means 104d, and a response generating means 803 generates voice, physical movement, expression, and shape change, which are appropriate to the input, to be output by an output means 102d. For a normal response, an input voice and a voice and movement to be responded are previously decided, for example, when the user utters "Good morning.," it replies "Good morning.," tilting its head, and when the user utters "I'm home.," it replies "Hello.," raising its hand.

Incorporating the judgment evaluation device of Embodiment 1 into such a pet-type terminal 800 allows, for example, the following use.

At the point when a predetermined time (for example, 30 minutes) passes while the pet-type terminal 800 and the user have a conversation, the response generating means 803, which receives a trigger signal from a timer means 801, generates a response for mode change. For example, it generates and outputs a response such as "Let's play a game."

When the voice recognition means 802 recognizes that the user made a positive reply such as "Yes." or "All right.," the response generating means 803 directs the question making means 101 to make a question. The question making means 101 makes a question, which is output.by the output means 102d. For the rest, similar operations are performed as in Embodiment 1, and, according to the result of the evaluation means 106, the pet-type terminal 800 notifies the result to the user by natural words such as "You are in good condition today." or "Are you tired a little bit?"

It is thought that animal therapy using pets such as dogs is effective for prevention of dementia and that contact with animals can provide mental relaxing, however, it has been indicated in the animal therapy that elderly people have difficulty in taking care of animals directly and that hygienic problems arise.

By having the structure of this Embodiment 5, quasi animal therapy using pet-type robots having the shapes of dogs, cats, and other various.animals can be applied to provide transition to a training mode through natural conversation with the pet-type robot, without requiring the user to do some special operation for training, so that training can be performed naturally without making the user conscious of the training.

The output means 102d corresponds to means including the question output means and the evaluation-result-information-notifying-to-outside means of the present invention, the input means 104d corresponds to means including the answer input means of the present invention, the judging means 105 corresponds to means including the determining means of the present invention, and the evaluation means 106 corresponds to means including the judgment evaluation means of the present invention. Also, the question composing means 109 and the timer means 801 correspond to means including the question making means of the present invention, and the correct answer making means 103 corresponds to means including the correct answer making means of the present invention. Also, the result recording means 107 corresponds to means' including the answer content recording means of the present invention. Also, the voice recognition means 802 corresponds to means including the input recognition means of the present invention, and the response generating means 803 corresponds to means including the response generating means and the condition changing means of the present invention. Also, means including the output means 102d, the input means 104d, the judging means 105, and the evaluation means 106 corresponds. to the judgment evaluation device of the present invention. Also, the pet-type terminal (intellectual judgment and mental function evaluation enabling pet-type robot) 800 corresponds to the robot of the present invention.

### (Embodiment 6)

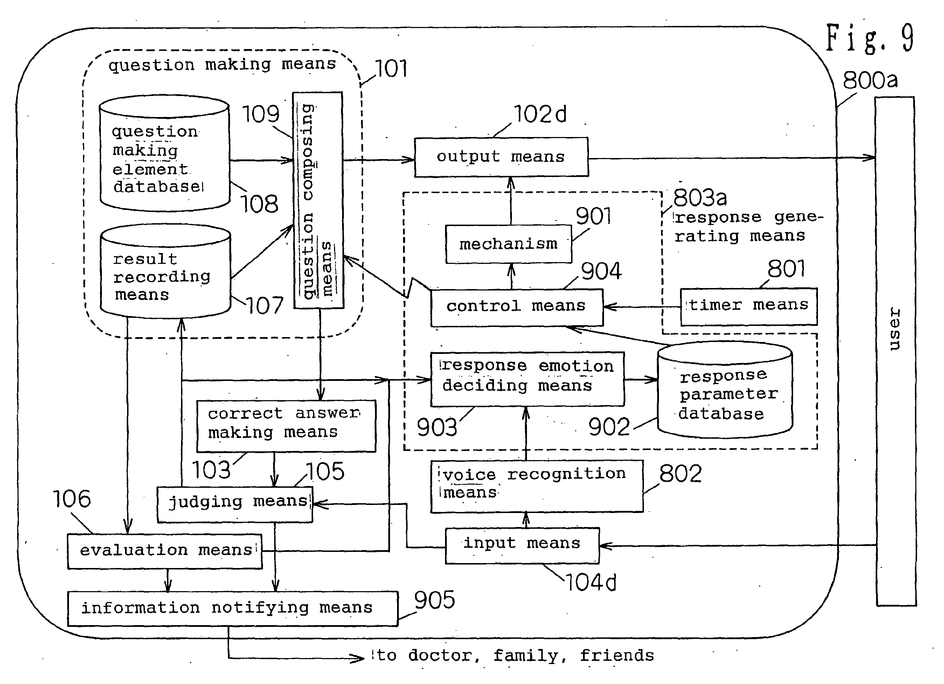

Next, referring mainly to Fig. 9, a configuration view of the pet-type terminal (intellectual judgment and mental function evaluation enabling pet-type robot) 800a of Embodiment 6 according to the present invention, the structure and operation of the pet-type terminal 800a of this embodiment is described. Means having the same numbers denoted to as in Fig. 8 provide the same operation as in Embodiment 5, and therefore their description is omitted.

A response generating means 803a comprises a mechanism 901, a response parameter database 902, a response emotion deciding means 903, and a control means 904, and generates a response according to the results of the judging means 105 and the evaluation means 106.

The generation of a response is described next. The mechanism 901 comprises a mechanism of opening and closing its eyelids and mouth, tilting its head, raising and lowering its arms, and the like, which are necessary for expressing face expression and body movement expression, and an actuator for actuation, and movement is generated by sending an appropriate control signal to the actuator. The parameter of a signal to be sent to the actuator of the mechanism 901 is recorded and held in the response parameter database 902 for each category of emotions of delight, anger, sorrow, and pleasure which are expressed by movements.

The response emotion deciding means 903 outputs a signal representing "delight" to the control means 904 when the result of the judging means 105 is a correct answer or when the result of the evaluation means 106 is "intellectual judgment is high", and outputs a signal representing "sorrow" or "anger" when the result of the judging means 105 is an incorrect answer or when the result of the evaluation means 106 is "intellectual judgment is low. " The control means 904 reads a signal for controlling the mechanism 901, which corresponds to the result of the response emotion deciding means 903, from the response parameter database 902, controls the mechanism 901, and generates a movement response.

As a result, when the evaluation of intellectual judgment is bad, the voice "Try harder a little bit.," which requires retraining, and sad expression are provided, and when the evaluation of intellectual judgment is good, the praising voice "Wow, great" and delight expression are provided, so that the user's motivation for training can be increased and continuous use can be expected.

Further, an information notifying means 905 periodically notifies the results of the judging means 105 and the evaluation means 106, information regarding the user's intellectual judgment, for example, changes in the correct answer percentage for each certain period, to the user's family and friends, his doctor and operator in the hospital and facilities. Thus, the sign of deterioration of brain function can be predicted in advance and quantitative evaluation can be performed, helping treatment and caring.

The output means 102d corresponds to means including the question output means of the present invention, the input means 104d corresponds to means including the answer input means of the present invention, the judging means 105 corresponds to means including the determining means of the present invention, and the evaluation means 106 corresponds to means including the judgment evaluation means of the present invention. Also, the question composing means 109 corresponds to means including the question making means of the present invention, and the question composing means 109 and the timer means 801 correspond to means including the correct answer making means of the present invention. Also, the result recording means 107 corresponds to means including the answer content recording means of the present invention. Also, the information notifying means 905 corresponds to the evaluation-result-information-notifying-to-outside means of the present invention. Also., the control means 904 corresponds to means including the condition changing means of the present invention. Also, means including the output means 102d, the input means 104d, the judging means 105, and the evaluation means 106 corresponds to the judgment evaluation device of the present invention. Also, the pet-type terminal (intellectual judgment and mental function evaluation enabling pet-type robot) 800a corresponds to the robot of the present invention.

### (Embodiment 7)

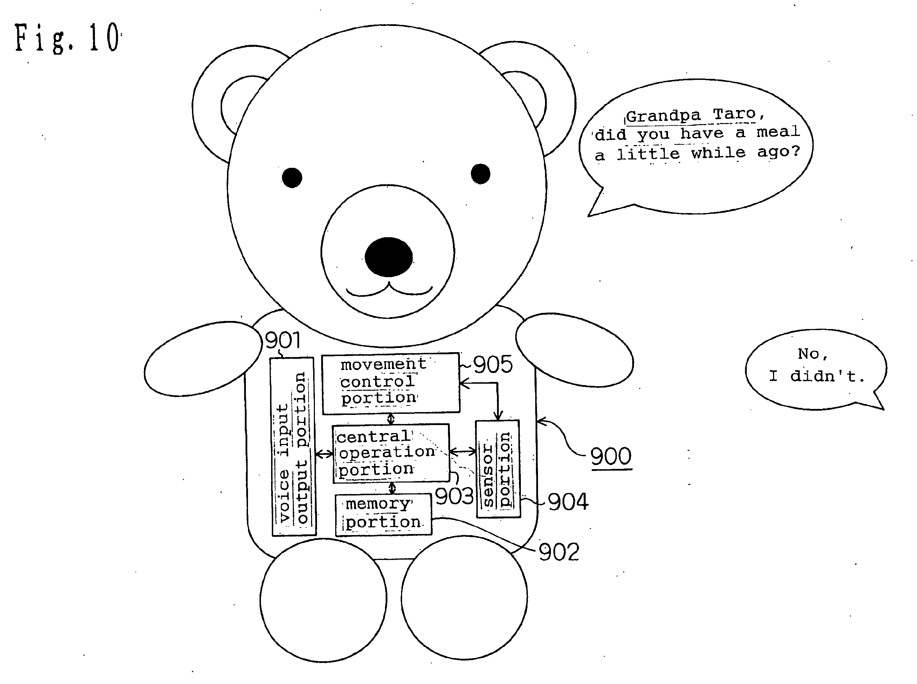

First, referring mainly to Fig. 10, a configuration view of the pet-type robot 900 of Embodiment 7 according to the present invention, the structure of the pet-type robot 900 of this embodiment is described.

A voice input output portion 901 is means having a microphone, a speaker, and the like, for voice-outputting the same question to the user for a plurality of times and voice-inputting the user's answers to the same question output for the plurality of times.

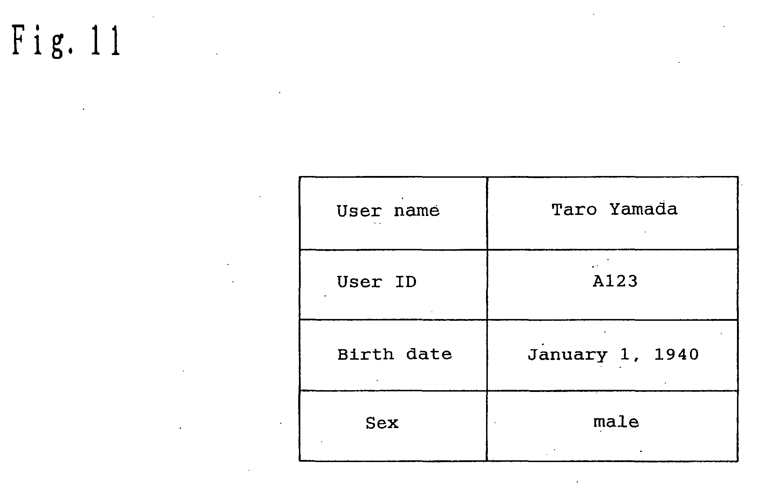

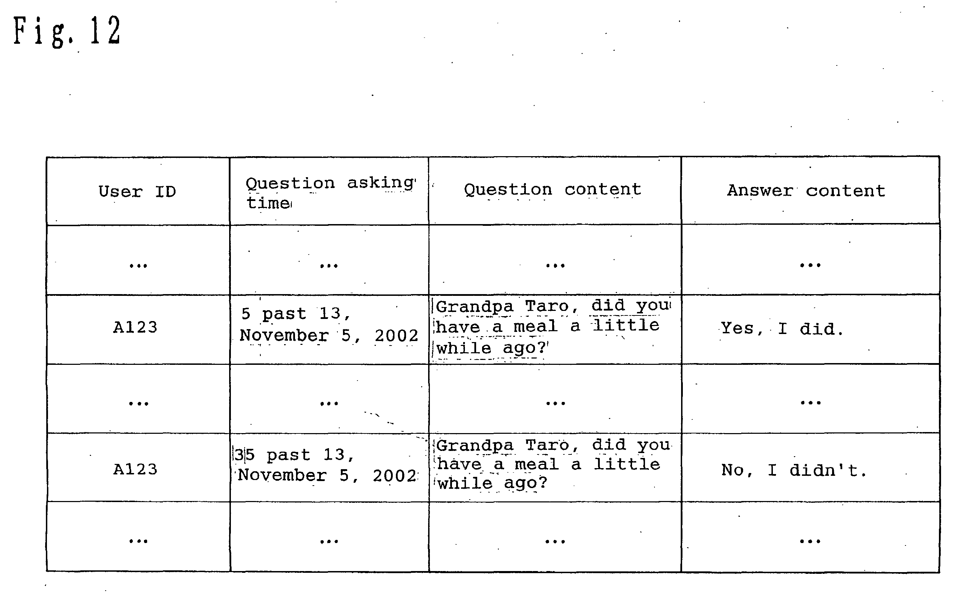

A memory portion 902 is means having a memory of holding (1) user voiceprint data for performing user person recognition, (2) user personal data regarding the user, which consists of four items of a user name, user ID, birth date, and sex as shown in Fig. 11, an explanation chart of the user personal data of Embodiment 7 according to the present invention, and (3) conversation history data regarding a question output for a plurality of times, the user's answers to the question, and the like, which consists of four items of a user ID, question asking times, questions, and answers as shown in Fig. 12, an explanation chart of the conversation history data of Embodiment 7 according to the present invention, and the like.

In this embodiment, the above user name, user ID, birth date, and sex are in turn "Taro Yamada," "A123," "January 1, 1940," and "male." Also, in this embodiment, the above user ID, question asking time, question, and answer are "A123," "5 past 13, November 5, 2000," "Grandpa Taro, did you have a meal a little while ago?," "Yes, I did.," and the like.

A central operation portion 903 is means having a CPU of performing an operation for evaluating the user's judgment with respect to the degree of dementia based on variation in the user's answers to the same question, and the like. Also, the central operation portion 903 is means of making a question to be output, using the user personal data, based on the result of user person recognition.

In this embodiment, the above questions are "Grandpa Taro, did you have a meal a little while ago?" and the like.

A sensor portion 904 is means having a sensor of sensing the presence of the user, and the like.

A movement control portion 905 is means having an actuator of changing utterance, expression, and gesture based on the foregoing evaluation result, and the like. Also, the movement control portion 905 is means of changing utterance, expression, and gesture based on the result of sensing as well when a question is output to the user.

Next, the operation of the pet-type robot 900 of this embodiment is described.

When the sensor portion 904 senses the presence of the user, the sensor portion 904 transmits it to the central operation portion 903.

When the central operation portion 903 receives the transmission from the sensor portion 904, it directs the movement control portion 905 to turn the body toward the direction in which the presence of the user is sensed, and directs the voice input output portion 901 to utter a cry.

When the movement control portion 905 receives the direction from the central operation portion 903, it turns the body toward the direction, in which the presence of the user is sensed, according to the direction. Also, when the voice input output portion 901 receives the direction from the central operation portion 903, it utters a cry according to the direction.

The user turns his body and notices the pet-type robot 900 that uttered the cry, then utters the voice "There, there."

When the voice input output portion 901 voice-inputs the voice "There, there" uttered by the user, the voice input output portion 901 transmits it to the central operation portion 903.

When the central operation portion 903 receives the transmission from the voice input output portion 901, it performs voice analysis using the user voiceprint data which is held in the memory portion 902, and recognizes that the user has the user personal data consisting of the content of the user name "Taro Yamada, " the user ID "A123," the birth date "January 1, 1940," and the sex "male."

Then, according to this recognition result, the central operation portion 903 makes the question "Grandpa Taro, did you have a meal a little while ago?" and directs the voice input output portion 901 to output the question made.

When the voice input output portion 901 receives the direction from the central operation portion 903, it voice-outputs the question "Grandpa Taro, did you have a meal a little while ago?" according to the direction.

When the user hears the question "Grandpa Taro, did you have a meal a little while ago?," he utters the voice "No, I didn't."

When the voice input output portion 901 voice-inputs the voice "No, I didn't" uttered by the user, the voice input output portion 901 transmits it to the central operation portion 903.

When the central operation portion 903 receives the transmission from the voice input output portion 901, it writes into the memory portion 902 conversation history data consisting of the content of the user ID "A123," the question asking time "35 past 13, November 5, 2002," the question "Grandpa Taro, did you have a meal a little while ago?," and the answer "No, I didn't."

Also, the central operation portion 903 looks into the memory portion 902 and recognizes that conversation history data, which consists of the content of the user ID "A123," the question asking time "5 past 13, November 5, 2002," the question "Grandpa Taro, did you have a meal a little while ago?," and the answer "Yes, I did.," is already written.

Then, the central operation portion 903 evaluates the user's judgment as being of a high degree of dementia, based on variation in the user's answers to the same question "Grandpa Taro, did you have a meal a little while ago?," which is output at an interval of only 30 minutes, makes the message "Grandpa Taro, are you all right?," and directs the voice input output portion 901 to output the message made.

When the voice input output portion 901 receives the direction from the central operation portion 903, it voice-outputs the message "Grandpa Taro, are you all right?" according to the direction.

Family and friends around this user can properly monitor the declining degree of the user's intellectual activity by listening to the message by such a pet-type robot 900.

The voice input output portion 901 corresponds to means including the question output means and the answer input means of the present invention, and the central operation portion 903 corresponds to means including the judgment evaluation means, the user person recognition means, and the question making means of the present invention. Also, the memory portion 902 corresponds to means including the user personal data holding means of the present invention. Also, the movement control portion 905 corresponds to means including the condition changing means of the present invention. Also, the sensor portion 904 corresponds to means including the user presence sensing means of the present invention. Also, the means including the voice input output portion 901 and the central operation portion 903 corresponds to means including the judgment evaluation device of the present invention. Also, the pet-type robot 900 corresponds to means including the robot of the present invention.

Also, while substantially the same question of the present invention is the same question "Grandpa Taro, did you have a meal a little while ago?" in this embodiment as described above, the question is not limited to this, for example, it may be a question having the same degree of difficulty or a question regarding similar content.

Thus far, these Embodiments 1-7 are described in detail.

The program of the invention is a program of causing a computer to perform the function of all or part of the means (or devices, elements, circuits, portions, or the like) of the judgment evaluation device or the robot of the present invention as described above, the program operating cooperatively with the computer. Also, the program of the present invention is a program of causing a computer to perform the operation of all or part of the steps (or processes, operations, actions, or the like) of the judgment evaluation method of the present invention as described above, the program operating cooperatively with the computer.

Also, the data structure of the invention is all or part of the means and steps of the judgment evaluation device, the robot, or the judgment evaluation method of the present invention as described above, the data structure being used cooperatively with a computer.

Also, the medium of the invention is a medium supporting a program of causing a computer to perform all or part of the functions of all or part of the means of the judgment evaluation device or the robot of the present invention as described above, the program, which is readable and is read by a computer, performing the functions cooperatively with the computer. Also, the medium of the present invention is a medium supporting a program of causing a computer to perform all or part of the operations of all or part of the steps of the judgment. evaluation method of the present invention as described above, the program, which is readable and is read by a computer, performing the operations cooperatively with the computer. Also, the medium of the invention is a medium supporting a data structure which is used cooperatively with a computer in all or part of the means and steps of the judgment evaluation device, the robot, or the judgment evaluation method of the present, invention as described above, the data structure, which is readable and is read by a computer, being used cooperatively with the computer.

The above "part of the means (or devices, elements, circuits, portions, or the like) " of the present invention and the above "part of the steps (or processes, operations, actions, or the like) of the present invention either mean, among a plurality of these means or steps, a number of means or steps, or mean part of the functions or operations of one means or step.

Also, the above "part of the devices (or elements, circuits, portions, or the like) " of the present invention mean, for example, a number of devices among a plurality of devices and the like which constitute the judgment evaluation device or the robot of the present invention, or mean part of the means (or elements, circuits, portions, or the like) of one device, or mean part of the functions of one means.

Also, in one use form of the program of the present invention, the program may be recorded in a recording medium, which is readable by a computer, and operate cooperatively with the computer. Also, in one use form of the program of the present invention, the program may be transmitted through a transmission medium, read by a computer, and operate cooperatively with the computer.

Also, the data structure of the invention includes a database, data format, data table, data list, data type, and the like.

Also, the recording medium includes ROM and the like, and the transmission medium includes a transmission medium such as the internet, light · radio waves · acoustic waves and the like.

Also, the computer of the present invention as described above is not limited to sheer hardware such as CPU and may include firmware, OS, and additional peripheral equipment.

As described above, the structure of the present invention may be implemented as software or may be implemented as hardware.

Thus, a simple interface is used to perform effective training for brain function using sight · hearing · touch. Further, by embedding the judgment evaluation device of the present invention in a pet-type terminal, training is carried out enjoyably through natural conversation and alone, so that no special operation is required and continuous use can be expected.

Also, the progress of intellectual and mental judgment deterioration can be prevented by using senses such as vision · hearing or using hand movement function and speech function while repeatedly answering to simple questions for brain function training for elderly people who suffer mild dementia or are in previous stages leading to dementia.

Also, the degree of progress of intellectual and mental judgment deterioration such as dementia is judged while intellectual training is unconsciously performed, and, further, these can readily be carried out, therefore continuous use can be achieved and deterioration of brain function can be prevented.

### Industrial Applicability

As is apparent from the above description, the present invention has the merit that the declining degree of intellectual activity can be monitored more properly, for example, without making a subject feel resistance to being subjected to judgment evaluation.

## Claims

1. A judgment evaluation device comprising:
a question output means of outputting a question to a user;
an answer input means of inputting an answer of the user to the output question;
a determining means of determining whether or not the input answer is correct; and
a judgment evaluation means of evaluating a judgment of the user based on a result of the determination.

2. The judgment evaluation device according to claim 1, further comprising a question making means of making the output question, and a correct answer making means of making a correct answer for the made question,
wherein the determining means determines whether or not the input answer is correct based on the made correct answer.

3. The judgment evaluation device according to claim 2, further comprising an answer content recording means of recording a content of the answer,
wherein the question making means makes the output question based on the recorded answer content.

4. The judgment evaluation device according to claim 2, wherein the question making means makes the question based on a result of the evaluation.

5. The judgment evaluation device according to claim 2, further comprising a sensor means of detecting a behavior of the user,
wherein the question making means makes the output question based on a result of the detection, and
the correct answer making means makes a correct answer for the made question based on the result of the detection.

6. The judgment evaluation device according to claim 2, further comprising an input recognition means of recognizing a predetermined input from the user, and a response generating means of generating a predetermined response to the user based on a result of the recognition,
wherein the question making means makes the output question based on the input recognition and/or the response generation.

7. The judgment evaluation device according to claim 1, wherein the evaluation means evaluates the judgment of the user by considering at least one of (1) date and time when the question is output to the user and/or date and time when the answer of the user to the question is input, and (2) a difficulty of the question output to the user.

8. The judgment evaluation device according to claim 1, wherein the question output means periodically outputs the question based on a predetermined standard.

9. The judgment evaluation device according to claim 1, wherein the question output means outputs the question using voice synthesis, and
the answer input means inputs the answer using voice recognition.

10. The judgment evaluation device according to claim 1, further comprising an evaluation-result-information-notifying-to-outside means of notifying information regarding a result of the evaluation to outside.

11. A judgment evaluation device comprising:
a question output means of outputting substantially the same question to a user for a plurality of times;
an answer input means of inputting an answer of the user to the substantially the same question output for the plurality of times; and
a judgment evaluation means of evaluating a judgment of the user based on variation in answers of the user to the substantially the same question.

12. The judgment evaluation device according to claim 11, wherein the evaluation is evaluation regarding a degree of dementia of the user.

13. The judgment evaluation device according to claim 12, further comprising:
a user personal data holding means of holding user personal data regarding a personal voice and/or personal image of the user;
a user person recognition means of performing user person recognition regarding the personal voice and/or personal image of the user; and
a question making means of making the output question using the held user personal data based on a result of the performed user person recognition.

14. A robot further comprising the judgment evaluation device according to any of claims 1 to 13; and
a condition changing means of changing a condition based on a result of the evaluation.

15. The robot according to claim 14, wherein the changing a condition is changing a condition regarding at least one of utterance, expression, and gesture.

16. The robot according to claim 15, further comprising a user presence sensing means of sensing a presence of the user,
wherein the condition changing means changes the condition based on a result of the sensing as well when a question is output to the user.

17. A judgment evaluation method comprising:
a question output step of outputting a question to a user;
an answer input step of inputting an answer of the user to the output question;
a determining step of determining whether or not the input answer is correct; and
a judgment evaluation step of evaluating a judgment of the user based on a result of the determination.

18. A judgment evaluation method comprising:
a question output step of outputting substantially the same question to a user for a plurality of times;
an answer input step of inputting an answer of the user to the substantially the same question output for the plurality of times; and
a judgment evaluation step of evaluating a judgment of the user based on variation in answers of the user to the substantially the same question.

19. A program of causing a computer to perform the question output step, the answer input step, the determining step, and the judgment evaluation step of the judgment evaluation method according to claim 17.

20. A program of causing a computer to perform the question output step, the answer input step, and the judgment evaluation step of the judgment evaluation method according to claim 18.

21. A medium supporting the program according to claim 19, which can be processed by a computer.

22. A medium supporting the program according to claim 20, which can be processed by a computer.
